# EUROPÄISCHE PATENTANMELDUNG

(11) **EP 0 897 908 A1**
(43) Veröffentlichungstag der Anmeldung: **24.02.1999**
(21) Anmeldenummer: 97114255.9
(22) Anmeldetag: 19.08.1997
(51) Int. Cl.: C07C 259/06, A61K 31/16

(54) **3-Aryl-Succinamido-Hydroxamsäuren, Prozesse zu ihrer Herstellung und diese Substanzen enthaltende Medikamente**

(71) Anmelder: BOEHRINGER MANNHEIM GMBH, 68298 Mannheim (DE)
(72) Erfinder: Grams, Frank Dr. Dipl.-Chemiker, 68219 Mannheim (DE); Zimmermann, Gerd Dr. Dipl.-Biologe, 76351 Linkenheim (DE); Krell, Hans-Willi Dr. Dipl.-Biologe, 82377 Penzberg (DE); Leinert, Herbert Dr. Dipl.-Chemiker, 64646 Heppenheim (DE); Menta, Ernesto Dr. Chemiker, 20063 Milano (IT)

(57) **Zusammenfassung**

Verbindungen der Formel I 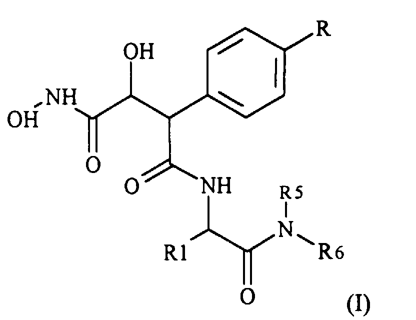 in denen
R, R1, R5 und R6 die Bedeutungen, wie in den Ansprüchen angegeben, besitzen deren pharmakologisch verträgliche Salze, Ester und Derivate, die zu in vivo zu Verbindungen der allgemeinen Formel I metabolisiert werden, sowie die Verwendung dieser Verbindungen zur Herstellung von Arzneimitteln.

## Beschreibung

Die vorliegende Anmeldung betrifft Inhibitoren der Matrixmetalloproteasen. Im speziellen betrifft sie 3-Aryl-succinamido-hydroxamsäuren.

Im gesunden Gewebe herrscht ein Gleichgewicht zwischen Synthese und Abbau der extrazellulären Matrix. Die extrazelluläre Matrix wird hauptsächlich durch Proteasen der Matrixmetalloprotease ("MMP") Familie degradiert. Beispiele für Mitglieder dieser Familie sind Collagenasen, Stromelysine und Gelatinasen. Im gesunden Gewebe ist dieser Abbau durch die Hemmung mit TIMPs ("tissue inhibitor of metalloproteases") reguliert. Dieses Gleichgewicht zwischen Matrixmetalloproteasen und TIMPs ist in verschiedenen Krankheiten wie Rheumatoider- und Osteoarthritis, Multiple Sclerosis, Tumor Metastasierung und -invasion, Cornea Ulcer, Meningitis, cardiovasculäre Erkrankungen wie Restenosis und Arteriosclerosis sowie Erkrankungen von Knochen und Zahnfleisch gestört. Viele Beispiele zeigen, daß eine Hemmung dieser Enzyme einen positiven Einfluß auf das klinische Bild dieser Krankheiten haben kann (Beckett et al., 1996).

Die wichtigsten MMPs für Metastasierung und Invasion scheinen die beiden Gelatinasen A (MMP-2) und B (MMP-9) zu sein. Eine selektive Hemmung dieser beiden Enzyme wäre wünschenswert. Die z.Z. am weitesten entwickelte Substanz in diesem Gebiet, Marimastat, die sich in Klinischer Phase III befindet, ist zwar aktiv, zeigt aber starke Nebenwirkungen wie Muskelschmerzen. Marimastat ist in der vorliegenden Form ein Breitband-MMP-Inhibitor, wodurch für den Gewebsstoffwechsel dringend notwendige MMPs wie die MMP1 ebenfalls gehemmt werden. Es wird allgemein angenommen, daß die Nebenwirkungen auf die Unspezifität zurückzuführen ist.

Überraschenderweise wurde nun gefunden, daß die neuen 3-Aryl-succinamidopropionohydroxamsäuren ein günstigeres pharmakologisches Profil als Marimastat zeigen. Diese Substanzen unterscheiden sich unter anderem von Marimastat durch die Arylsubstitution in Position 3 des Succinylrests. Die Spezifität gegenüber den Gelatinasen ist in den neuen Verbindungen deutlich verbessert, bzw. erst jetzt vorhanden. D h. die Hemmung der MMP1 und anderer wichtiger Enzyme kann vermieden werden.

Gegenstand der vorliegenden Anmeldung sind Substanzen der allgemeinen Formel I

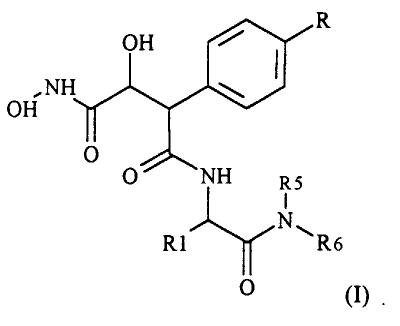

Um eine optimale Hemmung der Gelatinasen zu erreichen, sollen die Reste R und R1 der Verbindungen der allgemeinen Formel I gewisse Hydrophibizitäten aufweisen. Ein dafür geeigneter Parameter ist clogP, der mit Hilfe von 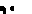 PCModels clogp3" von Daylight Chemical Information Systems, Inc (1993) bestimmt werden kann. Die Koeffizienten basieren auf Hansch, C. & Leo, A.: Substituent Constants for Correlation Analysis in Chemistry and Biology. Wiley Interscience New York (1979), während der Algorithmus auf folgender Referenzstelle basiert: Chou, J. & Jurs, P., J. Chem. Inf Comput. Sci. 19, 172 (1979). Die Fragmente werden zur Berechnung als vollständige Moleküle eingegeben, d.h. nicht als Radikal oder Ion. Um zu aussagekräftigen Werten zu kommen werden der clogP-Werte für R zusammen mit dem benachbarten Phenylring bestimmt (Ph-R-Fragmente). Die clogP-Werte des Restes R1 werden zusammen mit der benachbarten Carbonyl- und Aminofunktion bestimmt als (CHO)(NH₂)CHR1 (Aminocarbonyl-R1-Fragmente)

### Beispiele für Ph-R:

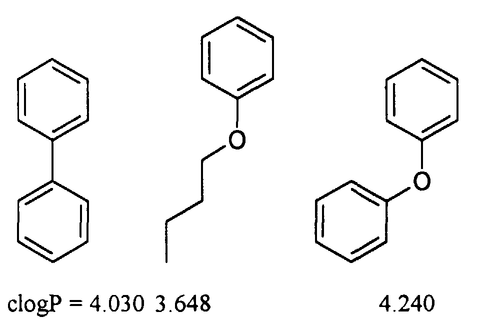

### Beispiele für Aminocarbonyl-R1:

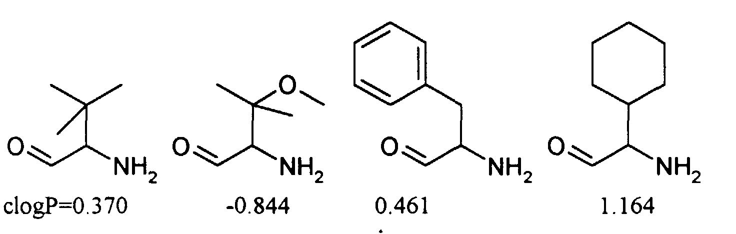

Die clogP-Werte für R in Form von 'Ph-R-Fragmente' bei den Verbindungen der vorliegenden Erfindung liegen bei 2.0 bis 6.0, bevorzugt bei 2.5 bis 5.0, besonders bevorzugt bei 3.0 bis 4.5.

Die clogP-Werte für R1 in Form von 'Aminocarbonyl-R1-Fragmente' bei den Verbindungen der vorliegenden Erfindung liegen zwischen -1.5 und 2.0, bevorzugt zwischen -1.2 und 1.5, besonders bevorzugt zwischen -1.0 und 1.2.

Gegenstand der vorliegenden Anmeldung sind daher Substanzen der allgemeinen Formel I 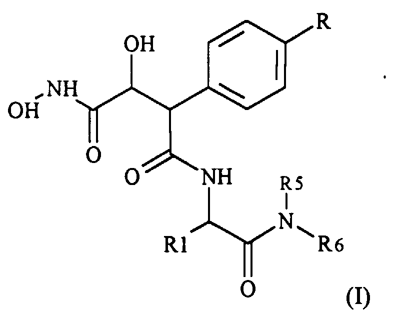 in denen
- R: als Ph-R-Fragment einen clogP-Wert zwischen 2.0 und 6.0, bevorzugt zwischen 2.5 und 5.0, besonders bevorzugt zwischen 3.0 und 4.5 aufweist,
- R1: als Aminocarbonyl-R1-Fragment einen clogP-Wert zwischen -1.5 und 2.0, bevorzugt zwischen -1.2 und 1.5, besonders bevorzugt zwischen -1.0 und 1.2 aufweist, und
- R5: Wasserstoff, oder einen C₁-C₈-AIkyl-Rest,
- R6: Wasserstoff, einen gegebenenfalls substituierten C₁-C₈-Alkyl-, oder einen gegebenenfalls substituierten monocyclischen oder bicyclischen Cycloalkyl-, Aryl-, Heteroaryl-, Aralkyl- oder Alkylheteroaryl-Rest,
oder
- R5 und R6: zusammen mit dem N-Atom einen gesättigten oder ungesättigten 5- oder 6-gliedrigen Ring, der maximal ein zusätzliches Heteroatom beinhaltet,
bedeutet,
deren pharmakologisch verträgliche Salze, Ester und Derivate, die zu in vivo zu Verbindungen der allgemeinen Formel I metabolisiert werden, sowie die Verwendung dieser Verbindungen zur Herstellung von Arzneimitteln.

Alternativ kann der Gegenstand der vorliegenden Anmeldung als Verbindungen der allgemeinen Formel I dargestellt werden: 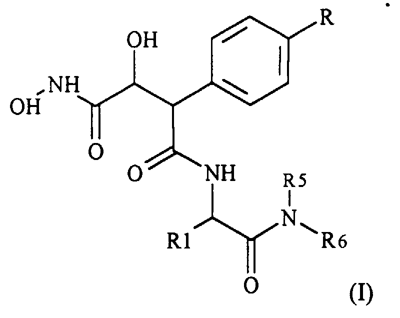 in denen
- R: einen gegebenenfalls substituierten C₁-C₈-Alkyl-, oder einen gegebenenfalls substituierten monocylischen oder bicyclischen Cycloalkyl, Aryl-, Heteroaryl-, Aryloxy-, Heteroaryloxy-, oder Aralkyl-Rest,
- R1: einen gegebenenfalls substituierten C₁-C₈-Alkyl-, oder einen gegebenenfalls substituierten monocyclischen oder bicyclischen Cycloalkyl-, Aryl-, Heteroaryl-, Aralkyl- oder Alkylheteroaryl-Rest,
- R5: Wasserstoff, oder einen C₁-C₈-Alkyl-Rest,
- R6: Wasserstoff, einen gegebenenfalls substituierten C₁-C₈-Alkyl-, oder einen gegebenenfalls substituierten monocyclischen oder bicyclischen Cycloalkyl-, Aryl-, Heteroaryl-, Aralkyl- oder Alkylheteroaryl-Rest,
oder
- R5 und R6: zusammen mit dem N-Atom einen gesättigten oder ungesättigten 5- oder 6-gliedrigen Ring, der maximal ein zusätzliches Heteroatom beinhaltet,
bedeutet,
deren pharmakologisch verträgliche Salze, Ester und Derivate, die zu in vivo zu Verbindungen der allgemeinen Formel I metabolisiert werden, sowie die Verwendung dieser Verbindungen zur Herstellung von Arzneimitteln.

Die unter R, R1 und R6 aufgeführten Reste können unabhängig voneinander gegebenenfalls durch Halogen, Hydroxy, Thio, Alkyl, C₃-C₈-Cycloalkyl, C₄-C₈-Cycloalkenyl, Hydroxyalkyl, Alkoxy, Hydroxyalkoxy, Chloralkoxy, Alkylthio, Alkylsulfinyl, Alkylsulfonyl, Amino, Alkylamino, Dialkylamino, Nitro, Carboxyl, Carboxamido, Alkoxycarbonyl, Alkoxycarbonylalkyl, Perfluoralkyl, gegebenenfalls ein- oder zweifach durch niederes Alkyl substituiertes Amino bzw. Aminocarbonyl, Nitril, Oxo, oder Acyl ein-, zwei oder dreifach substituiert sein

Bevorzugt sind hierbei vor allem die Halogen, Hydroxy, Oxo, Thio, Alkoxy, ω-Hydroxyalkoxy, ω-Chloralkoxy, Alkylthio, Amino, Aminocarbonyl, Carboxyl, und Acylgruppe. Besonders bevorzugt ist Methyl, Halogenyl und Hydroxyl.

Wenn substituiert ist, ist die einfache Substitution bevorzugt.

Cycloalkyl bedeutet, wenn nicht anders angegeben ein gesättigter, ein- oder mehrfach ungesättigten Carbocyclus oder Heterocyclus mit 3 bis 8 Gerüstatomen, vorzugsweise 5 - 7 Gerüstatomen, die gegebenenfalls ein- oder mehrfach durch Heteroatome wie Stickstoff Sauerstoff oder Schwefel unterbrochen sein können, insbesondere den Cyclopentyl-, Cyclohexyl-, Cycloheptyl-, Morpholinyl-, Thiamorpholinyl-, Piperidinyl-, Piperazinyl-, Tetrahydrofuranyl-, Tetrahydropyranyl-Rest.

Der Alkylrest in Aralkyl und Heteroalkyl in R, R1 und R6 bedeutet unabhängig voneinander C₁- oder C₂-Alkyl.

Acyl bei den Resten R, R1 und R6 bedeutet vor allem die Acetylgruppe.

Alkyl bei den Resten R, R1 und R6 bedeutet, wenn nicht anders angeben, für sich oder in Kombination z. B. mit Alkoxy, Alkylthio, Arylsulfonyl, Alkylsulfonyl, Alkylaminocarbonyl, Arylaminocarbonyl, Alkylamino, Alkoxycarbonyl, Aryloxycarbonyl, Alkylaminothiocarbonyl, Arylaminothiocarbonyl, einen geradkettigen, verzweigten, gesättigten oder ungesättigten Rest mit 1 - 3 Kohlenstoffatomen wie z. B. den Methyl, Ethyl, Propyl, Isopropyl, Allyl oder Propinyl.

C₁-C₈-Alkyl bei den Resten R, R1, R5 und R6 bedeutet unabhängig voneinander einen geradkettigen, verzweigten, gesättigten oder ungesättigten Rest mit 1 - 8 Kohlenstoffatomen, der durch 1 oder 2 Heteroatome wie O, N oder S unterbrochen sein kann, wobei keine Unterbrechung oder eine Unterbrechung durch Sauerstoff bevorzugt ist, wobei bei Sauerstoffunterbrechung besonders C₁-C₇-Alkoxy bevorzugt ist. Beispiele für C₁-C₈-Alkylreste sind Methyl, Ethyl, Propyl, Pentyl, Octyl, Allyl, Propargyl, 2,4-Pentadienyl, Isopropyl, sec. Butyl, tert. Butyl, 3-Methyl-butyl, 2-Hydroxy-hexyl, n-Butoxy, Hexyloxy, C(CH₃)₂OMe, insbesondere tert. Butyl, n-Butoxy, Hexyloxy, C(CH₃)₂OMe.

Unter Aryl versteht man den Phenyl- oder Naphthylrest, die gegebenenfalls substituiert sein können, insbesondere durch Halogen, Alkyl oder Alkoxy. Bevorzugt ist der Phenylrest.

Unter Halogen versteht man Chlor, Brom oder Jod, vorzugsweise Chlor.

Unter Gerüstatomen versteht man C, N, O und S, unter Heteroatomen N, O und S.

Die bei R, R1 und R₆ aufgeführten Heteroarylreste bedeuten unabhängig voneinander einen Pyridin-, Pyrimidin, Pyridazin, Pyrazin-, Piperazin-, Imidazol-, Furan, Oxazol, Isothiazol, Isoxazol, 1,2,3-Triazol- oder 1,2,4-Triazol, Thiazol-, Thiophen- oder Indolring, vorzugsweise den Pyridin-, Imidazol- und Thiophenring.

Bei dem unter R, R₁ und R₆ aufgeführten Bicyclen handelt es sich vorzugsweise um Reste wie den Naphthyl-, Tetrahydronaphthyl-, Dekalinyl-, Chinolinyl-, Isochinolinyl-, Tetrahydrochinolinyl-, Tetrahydroisochinolinyl-, Indolyl-, Benzimidazolyl-, Indazolyl-, Oxindolyl, Benzofuranyl-, Benzothiophenyl-, Benzthiazolyl, Benzoxazolyl- oder den Purinylrest, insbesondere aber um den Naphthyl-, Chinolinyl-, Isochinolinyl-, Tetrahydrochinolinyl-, Indolyl-, oder Benzimidazolylrest.

Bevorzugte Reste für R sind 4 bis 7 Gerüstatome für Alkyl und Cycloalkyl, für die Aryl, Bicyclus und Heteroarylreste sind es 5 bis 10, besonders bevorzugt 6 bis 8 Gerüstatome.

Bevorzugte Reste für R1 sind 1 bis 12 Gerüstatome, besonders bevorzugt 3 bis 10 Gerüstatome.

Ganz besonders bevorzugte Reste für R sind: -O-Phenyl(p-R2), -Phenyl(p-R2), n-Butoxy, Hexyloxy, wobei R2 für einen kleinen Substituenten wie Wasserstoff, Halogenyl, Methyl oder Hydroxyl steht, insbesondere bevorzugte Reste für R sind -O-Phenyl, -Phenyl und n-Butoxy.

Ganz besonders bevorzugt für R1 sind die Reste Benzyl, Phenyl, tert-Butyl oder C(CH₃)₂OMe, dabei insbesondere tert-Butyl.

Bevorzugte Reste für R5 sind Wasserstoff, Methyl und Ethyl, besonders bevorzugt ist Wasserstoff.

Bevorzugte Reste für R6 sind Methyl und Ethyl, Phenyl und Pyridyl, besonders bevorzugt ist Methyl

Bilden R5 und R6 einen Zyklus enthält dieser bevorzugt Sauerstoff besonders bevorzugt ist Morpholino.

Die Erfindung betrifft außerdem alle optischen Isomere sowie die Racemate. Bevorzugt sind folgende optische Isomere: C2 der Bernsteinhydroxamsäure und Cα des Aminosäureamids in S-Konfiguration und C3 des Bernsteinsäuregrundkörpers in R Konfiguration.

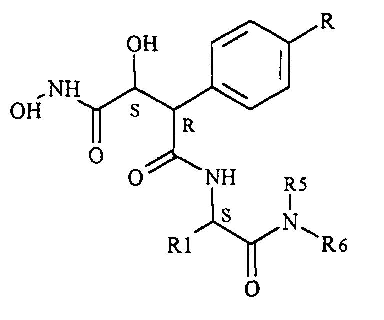

Die erfindungsgemäßen Verbindungen der allgemeinen Formel I lassen sich nach bekannten Verfahren synthetisieren. Die optischen Isomere können nach an sich bekannten Methoden getrennt werden. Die beschriebenen Verfahren beziehen sich, wo immer sinnvoll auf Trennung von Endstufen und/oder Vorstufen. Entweder werden aus den racemischen Gemischen durch Umsetzung mit einer optisch aktiven Säure wie z. B. D- oder L-Weinsäure, Mandelsäure, Äpfelsäure, Milchsäure oder Camphersulfonsäure bzw. einem optisch aktiven Amin wie z. B. D- oder L-α-Phenylethylamin, Ephedrin, Chinidin oder Cinchonidin diastereomere Salze gebildet, die durch Kristallisation getrennt werden können, oder die Optischen Isomere werden per HPLC getrennt. Eine weitere Möglichkeit optische Isomere zu trennen besteht während der Synthese in einer enzymatischen Trennung und/oder Synthese der Zwischenprodukte.

Die Verbindungen der allgemeinen Formel I sind erhältlich durch
(a) Umsetzung einer Carbonsäure der allgemeinen Formel II, in der R, R1, R5 und R6 die oben genannte Bedeutung hat und in der die alkoholische Hydroxygruppe frei oder auch geschützt z. B. durch Esterbildung mit Essigsäure vorliegen kann 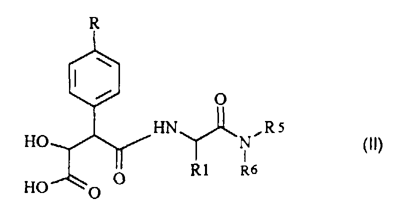 mit Hydroxylamin oder einem an O- oder N,O-geschützten Hydroxylamin mit anschließender Abspaltung der Schutzgruppe.
   Für diese Umsetzungen lassen sich Carbonsäuren nach den aus der Peptidchemie bekannten Verfahren aktivieren. Beispielsweise kann man Carbonsäuren durch Umsetzung mit Chlorameisensäureestern, Carbodiimiden, N,N'-Carbonyldiimidazol, 2-Chlor-N-methylpyridiniumjodid direkt aktivieren, oder intermediär zu aktiven Estern wie Pentafluorphenyl-, N-Hydroxysuccinimid, N-Hydroxybenzotriazolestern umsetzen, die dann mit Hydroxylamin oder einem geschützten Hydroxylamin zur Reaktion gebracht werden können. Nach vollendeter Umsetzung eines geschützten Hydroxylaminderivates werden die Schutzgruppen nach bekannten Methoden abgespalten. Beispiel für geschützte Hydroxylamine sind O-Benzylhydroxylamin, O-p-Methoxybenzylhydroxylamin, O-Trimethylsilylhydroxylamin und O-tert-Butylhydroxylamin, N,O-Bisbenzylhydroxylamin, N,O-Bis-p-methoxybenzylhydroxylamin. Die Abspaltung der Schutzgruppen kann bei Benzyl- oder p-Methoxybenzylgruppen durch Hydrogenolyse oder im letzteren Fall und bei der O-tert-Butylgruppe auch durch saure Hydrolyse erfolgen. Die Trimethylsilylschutzgruppe läßt sich durch Wasser hydrolysieren.
(b) alternativ zu (a) kann man die Verbindungen der Formel I durch Umsetzung eines 1,3-Dioxolan-4-ons der allgemeinen Formel III, in der R₃ und R₄ für Wasserstoff, Niederalkyl oder Phenyl, bevorzugt Methyl, stehen, durch Umsetzung mit Hydroxylamin herstellen.

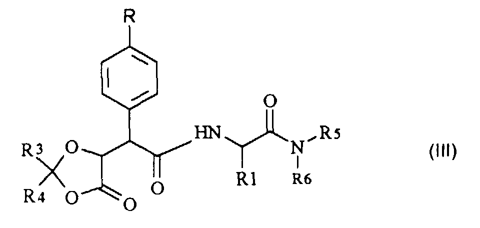

Die Verbindungen der allgemeinen Struktur II lassen sich durch alkalische Hydrolyse von Verbindungen der Struktur III herstellen.

Die Verbindungen der allgemeinen Struktur III sind erhältlich durch Kupplung einer 2-Aryl-3-hydroxy-bernsteinsäure der allgemeinen Struktur IVa, in der R, R3 und R4 die oben genannte Bedeutung besitzen und deren Hydroxygruppe und die dazu benachbarte COOH-Gruppe unter Bildung einer 1,3-Dioxolan-4-onstruktur geschützt sind, 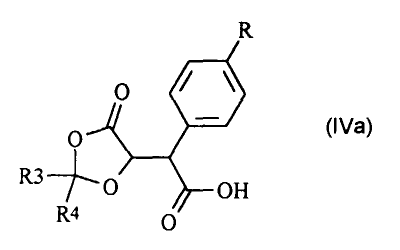 mit racemischen oder optisch einheitlichen α-Aminosäuren z. B. (R)- oder (S)-tert-Butyl-glycin-N-methylamid nach Methoden, die aus der Peptidchemie bekannt sind. Beispielsweise lassen sich durch Aktivierung der COOH-Gruppe in IVa durch Carbodiimide wie Di-cyclohexylcarbodiimid oder Di-isopropylcarbodiimid, aktive Ester wie z. B. N-Hydroxysuccinimid-, 1-Hydroxybenzotriazol- oder Pentafluorphenylester herstellen, die dann mit der freien Aminogruppe eines an der Amidgruppe substituierten α-Aminosäure-amids reagieren können. Diese Ester können auch ohne Isolierung hergestellt und weiter umgesetzt werden. Andere Aktivierungsmethoden umfassen die Herstellung von gemischten Anhydriden durch Umsetzung der Carbonsäuren mit Chlorameisensäureestern oder Kondensationsreagentien wie Uroniumsalze z.B. 2H-(1H-Benzotriazol-1-yl)-1,1,3,3,-tetramethyluronium-tetrafluoroborat.

Die Carbonsäuren der Formel IVa lassen sich durch Umsetzung von α-Formylarylessigsäuren der allgemeinen Formel V, 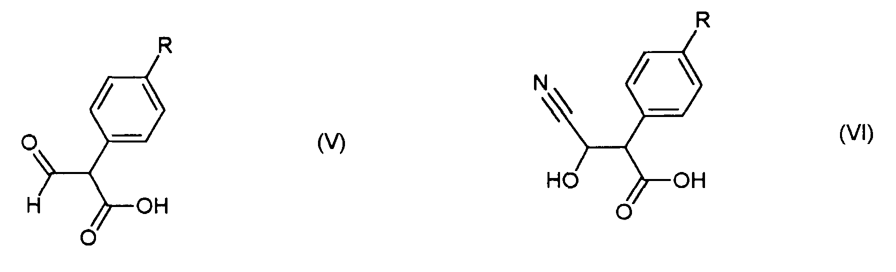 in der R die oben genannte Bedeutung hat, mit Alkalicyaniden oder Trimethylsilylcyanid/Zinkiodid, saurer Verseifung der als Zwischenprodukte erhaltenen Cyanhydrine der allgemeinen Struktur VI und anschließendem Schutz der Hydroxy- und der COOH-Gruppe durch eine sauer katalysierte Reaktion mit Acetalen oder Ketalen, z. B. 2,2-Dimethoxypropan herstellen. Die Cyanhydrine lassen sich auch optisch aktiv durch enzymatische Umsetzung der Verbindung der allgemeinen Struktur V unter Katalyse durch (R)- oder (S)-Oxynitrilasen, die jeweils stereospezifisch die R- bzw. S-Verbindung ergeben, herstellen.

Alternativ kann man Verbindungen der Formel IVa durch Reduktion von Oxalarylessigsäurestern z. B. mit Natriumborhydrid, saurer Verseifung der Ester und anschließendem Schutz der Hydroxygruppe und der benachbarten COOH-Gruppe durch eine sauer katalysierte Reaktion mit Acetalen oder Ketalen z. B. 2,2-Dimethoxypropan herstellen.

Als pharmakologische verträgliche Salze werden vor allem Alkalisalze, Ammoniumsalze, Acetate oder Hydrochloride verwendet, die man in üblicher Weise z. B. durch Titration der Verbindungen mit anorganischen oder organischen Basen oder anorganischen Säuren wie z. B. Natriumhydroxyd, Kaliumhydroxyd, wäßrigem Ammoniak, Aminen wie z. B. Triethylamin oder Salzsäure herstellt. Die Salze werden in der Regel durch Umfällen aus Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspension etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z. B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z. b. Stärke, Lactose, Mannit, Methylcellulose, Talkum, hochdisperse Kieselsäuren, höhermolekulare Fettsäuren (wie Stearinsäure), Gelantine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 10-1000 mg/Mensch, vorzugsweise bei 100-500 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden 3-Arylsuccinamo-hydroxamsäurederivate:
1. 2(Biphenyl-4-ylamino)-N1-(2,2-dimethyl-1-methylcarbamoyl-propyl)3,N4-dihydroxy-succinamid
2. 2(Biphenyl-4-ylamino)-N1-[2,2dimethyl-1-(morpholine-4-carbonyl)propyl]-3,N4-dihydroxy-succinamid
3. 2(Biphenyl-4-ylamino)-N1-[2,2-dimethyl-1-(pyridin-2-ylcarbamoyl)-propyl]-3,N4-dihydroxy-succinamid
4. 2-(Biphenyl-4-ylamino)-3,N4-dihydroxy-N1-(1-methylcarbamoyl-2-phenyl-ethyl)-succinamid
5. N1-(1-Benzyl-2-morpholin-4-yl-2-oxo-ethyl)-2-(biphenyl-4-ylamino)-3,N4-dihydroxy-succinamid
6. 2(Biphenyl-4-ylamino)-3,N4dihydroxy-N1-[2-phenyl-1-(pyridin-2-ylcarbamoyl)-ethyl]succinamid
7. 2-(Biphenyl-4-ylamino)3,N4-dihydroxy-N1-(2-methoxy-2-methyl-1-methylcarbamoyl-propyl)-succinamid
8. 2(Biphenyl-4-ylamino)-3,N4-dihydroxy-N1-[2-methoxy-2-methyl-1-(morpholine-4-carbonyl)-propyl]-succinamid
9. 2-(Biphenyl-4-ylamino)-3,N4-dihydroxy-N1-[2-methoxy-2-methyl-1-(pyridin-2-ylcarbamoyl)-propyl]-succinamid
10. N4-(2,2-Dimethy-1-methylcarbamoyl-propyl)-2,N1-dihydroxy-3-(4-phenoxyphenylamino)-succinamid
11. N4-[2,2-Dimethyl-1-(morpholine-4-carbonyl)-propyl]-2,N1-dihydroxy-3-(4-phenoxy-phenylamino)-succinamid
12. N4-[2,2-Dimethyl-1-(pyridin-2-ylcarbamoyl)-propyl]-2,N1-dihydroxy-3-(4-phenoxyphenylamino)succinamid
13. 2,N1-Dihydroxy-N4-(1-methylcarbamoyl-2-phenyl-ethyl)-3-(4-phenoxyphenylamino)-succinamid
14. N4-(1-Benzyl-2-morpholin-4yl-2-oxo-ethyl)-2,N1-dihydroxy-3-(4-phenoxyphenylamino)-succinamid
15. 2,N1-Dihydroxy-3-(4-phenoxy-phenylamino)-N4-[2-phenyl-1-(pyridin-2-ylcarbamoyl)-ethyl]-succinamid
16. 2,N1-Dihydroxy-N4-(2-methoxy-2-methyl-1-methylcarbamoyl-propyl)-3-(4-phenoxy-phenylamino)-succinamid
17. 2,N1-Dihydroxy-N4-[2-methoxy-2-methyl-1(morpholine-4-carbonyl)-propyl]-3-(4-phenoxy-phenylamino)-succinamid
18. 2,N1-Dihydroxy-N4-[2-methoxy-2-methyl-1-(pyridin-2-ylcarbamoyl)-propyl]-3-(4-phenoxy-phenylamino)-succinamid
19. 2-(4-Butoxy-phenylamino)-N1-(2,2-dimethyl-1-methylcarbamoyl-propyl)-3,N4-dihydroxy-succinamid
20. 2(4-Butoxy-phenylamino)-N1-[2,2-dimethyl-1-(morpholine-4-carbonyl)-propyl]-3,N4-dihydroxy-succinamid
21. 2-(4-Butoxy-phenylamino)-N1-[2,2-dimethyl-1-(pyridin-2-ylcarbamoyl)-propyl]-3,N4-dihydroxy-succinamid
22. 2-(4-Butoxy-phenylamino)-3,N4-dihydroxy-N1-(1-methylcarbamoyl-2-phenylethyl)-succinamid
23. N1-(1-Benzyl-2-morpholin-4-yl-2-oxo-ethyl)-2-(4-butoxy-phenylamino)-3,N4-dihydroxy-succinamid
24. 2-(4-Butoxyphenylamino)-3,N4-dihydroxy-N1-[2-phenyl-1-(pyridin-2-ylcarbamoyl)ethyl]-succinamid
25. 2-(4-Butoxy-phenylamino)-3,N4-dihydroxy-N1-(2-methoxy-2-methyl-1-methylcarbamoylpropyl)-succinamid
26. 2-(4-Butoxy-phenylamino)-3,N4-dihydroxy-N1-[2-methoxy-2-methyl-1-(morpholine-4-carbonyl)-propyl]-succinamid
27. 2-(4-Butoxy-phenylamino)3,N4-dihydroxy-N1-[2-methoxy-2-methyl-1-(pyridin-2-ylcarbamoyl)-propyl]-succinamid
28. N1-(2,2-Dimethyl-1-methylcarbamoyl-propyl)-2-(4-hexyloxy-phenylamino)-3,N4-dihydroxy-succinamid
29. N1-[2,2-Dimethyl-1-(morpholine-4-carbonyl)-propyl]-2-(4-hexyloxyphenylamino)3,N4-dihydroxy-succinamid
30. N1-[2,2-Dimethyl-1-(pyridin-2-ylcarbamoyl)-propyl]-2-(4-hexyloxy phenylamino)3,N4-dihydroxy-succinamid
31. 2-(4-Hexyloxy-phenylamino)-3,N4-dihydroxy-N1 -(1-methylcarbamoyl-2-phenylethyl)-succinamid
32. N1-(1-Benzyl-2-morpholin-4-yl-2-oxo-ethyl)-2-(4-hexyloxy-phenylamino)-3,N4-dihydroxy-succinamid
33. 2-(4-Hexyloxy-phenylamino)-3,N4-dihydroxy-N1-[2-phenyl-1-(pyridin-2-ylcarbamoyl)-ethyl]-succinamid
34. 2-(4-Hexyloxy-phenylamino)-3,N4-dihydroxy-N1-(2-methoxy-2-methyl-1-methylcarbamoyl-propyl)-succinamid
35. 2-(4-Hexyloxy-phenylamino)-3,N4-dihydroxy-N1-[2-methoxy-2-methyl-1-(morpholine-4-carbonyl)-propyl]-succinamid
36. 2-(4-Hexyloxy-phenylamino)-3,N4-dihydroxy-N1-[2-methoxy-2-methyl-1-(pyridin-2-ylcarbamoyl)-propyl]-succinamid

### Beispiel 1

### 2-Biphenyl-4-yl-N1-((1S)-2,2-dimethyl-1-methylcarbamoyl-propyl)-3,N4-dihydroxy-succinamid

### 1.1. α-Formyl-biphenylessigsäure-ethylester

Eine Mischung aus 24 g Biphenylessigsäureethylester und 8.2 g Ameisensäureethylester wird tropfenweise unter Rühren zu einer Suspension von 2.4 g Natriumhydrid in 250 ml Ether gegeben. Das Reaktionsgemisch wird 2 Stunden bei Raumtemperatur gerührt und vorsichtig mit kaltem Wasser versetzt. Die wässrige Phase wird auf pH 2 angesäuert und das Produkt mit Ether extrahiert. Der Extrakt wird getrocknet und eingedampft.

### 1.2. 2-Biphenyl-3-hydroxy-bernsteinsäure

Eine Lösung von 5.7 g α-Formyl-biphenylessigsäure-ethylester in 70 ml Dichlormethan wird mit 2 g Trimethylsilylcyanid und einer katalytischen Menge Zinkiodid versetzt. Die Mischung wird über Nacht bei Zimmertemperatur gerührt und eingedampft. Der Rückstand wird mit 6 N HCl versetzt und zur Verseifung der Nitril-, Trimethylsilyl- und der Estergruppe unter Rückfluß gekocht. Das Produkt wird durch Extraktion mit Ether isoliert.

### 1.3. α-2,2-Dimethyl-1,3-dioxolan-4-on-5-yl-biphenylessigsäure

3.14 g 2-Biphenyl-3-hydroxy-bernsteinsäure werden in 100 ml 2,2-Dimethoxypropan und 30 ml DMF gelöst, mit einer katalytischen Menge p-Toluolsulfonsäure versetzt und über Nacht bei 30 - 40 Grad gerührt. Das Lösungsmittel wird abgedampft und das Rohprodukt in der nächsten Stufe weiterverwendet.

### 1.4. α-2,2-Dimethyl-1,3-dioxolan-4-on-5-yl-biphenylacetyl-(S)-tert-glycin-N'-methylamid

0.6 g α-2,2-Dimethyl-1,3-dioxolan-4-on-5-yl-biphenylessigsäure und 0.3 g (S)-tert.-Glycin-N'-methylamid werden in 30 ml Methylenchlorid gelöst und mit 0.2 g Diisopropyl-ethylamin versetzt. Dazu gibt man 0.5 g 2H-(1H-Benzotriazol-1yl)-1,1,3,3,-tetramethyluronium-tetrafluoroborat und rührt das Gemisch über Nacht. Man versetzt mit etwa 200 ml Essigester und wäscht die organische Phase nacheinander mit Natriumbicarbonatlösung, 0.5 N Salzsäure und erneut mit Natriumbicarbonatlösung. Die organische Phase wird getrocknet und eingedampft und der Rückstand mit Ether verrieben.

### 1.5. 2-Biphenyl-4-yl-N1-((1S)2,2-dimethyl-1-methylcarbamoyl-propyl)-3,N4-dihydroxy-succinamid

Zu einer Lösung von 0.14 g Hydroxylamin-Hydrochlorid in Methanol werden 0.14 g Natriummethylat zugegeben und die Mischung zwei Stunden bei Raumtemperatur gerührt. Der ausgefallene Niederschlag wird abfiltriert und das Filtrat im Eisbad abgekühlt. Dazu werden portionsweise 0.9 g des oben erhaltenen R,S-α- 2,2-Dimethyl-1,3-dioxolan-4-on-5-yl-biphenylacetyl-tert-glycin-N'-methylamids hinzugegeben. Die Mischung wird 30 min bei 0 Grad und dann bei Zimmertemperatur über Nacht gerührt. Die Reaktionslösung wird eingedampft und der Rückstand durch Chromatographie an Kieselgel mit Methylenchlorid/Methanol (3 - 10 %) als Eluent aufgereinigt.

### Beispiel 2

Um das Inhibierungspotential an MMPs, z.B. HNC, der Verbindungen der allgemeinen Formel I festzustellen wird die katalytische Domäne (Isolation und Reinigung siehe Schnierer, S., Kleine, T., Gote, T., Hillemann, A., Knäuper, V., Tschesche, H., Biochem. Biophys. Res. Commun. (1993) 191, 319-326) inkubiert. Anschließend wird die anfangliche Reaktionsrate eines Standardsubstrates gemessen, wie in Grams F. et al., FEBS 335 (1993) 76-80). Die Ergebnisse werden in üblicher Weise ausgewertet (s. z.B. Dixon, M., Biochem. J. (1953) 55, 170-202.)

Das synthetische Kollagenase Substrat ist ein C-terminal mit Dinitrophenol (DNP) gekoppeltes Heptapeptid. Dieser DNP Rest quencht die Fluoreszenz des Trp des Heptapeptides. Nach der Spaltung eines Tripeptids mit dem DNP Rest, nimmt die meßbare Fluoreszenz zu.
Assay Puffer:
   50 mM Tris/HCl pH 7.6 (Tris = Tris-(hydroxymethyl)-aminomethan)
   100 mM NaCl/10 mM CaCl₂/5 % MeOH (wenn nötig)
Enzym: 8 nM katalytische Domäne (Met80-Gly242) der menschlichen Neutrophilen Kollagenase
Substrat: 10 µM DNP-Pro-Leu-Gly-Leu-Trp-Ala-D-Arg-NH2
Gesamtassayvolumen: 1 ml

Es wird eine Lösung aus Enzyme und Inhibitor in Assaypuffer bei 25 °C hergestellt. Die Reaktion wird durch Zugabe des Substrats in die Lösung gestartet. Die Spaltung des fluorogenen Substrats wird mit Fluoreszenz Spektroskopie gemessen (Anregungs- bzw. Emissionswellenlänge bei 280 und 350 nm). Der IC₅₀ Wert wird festgelegt für eine Inhibitorkonzentration bei der die Reaktionsgeschwindigkeit halbiert wird.

Die Verbindungen der allgemeinen Formel I wirken als Inhibitoren.

## Patentansprüche

1. Verbindungen der Formel I 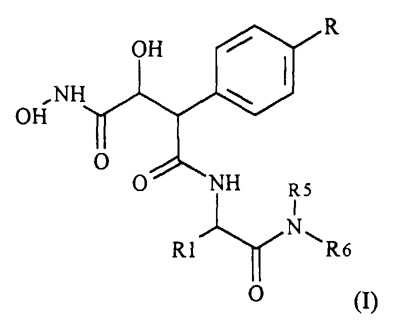 in denen
R einen gegebenenfalls substituierten C₁-C₈-Alkyl-, oder einen gegebenenfalls substituierten monocylischen oder bicyclischen Cycloalkyl-, Aryl-, Heteroaryl-, Aryloxy-, Heteroaryloxy-, oder Aralkyl-Rest,
R1 einen gegebenenfalls substituierten C₁-C₈-Alkyl-, oder einen gegebenenfalls substituierten monocyclischen oder bicyclischen Cycloalkyl-, Aryl-, Heteroaryl-, Aralkyl- oder Alkylheteroaryl-Rest,
R5 Wasserstoff oder einen C₁-C₈-Alkyl-Rest,
R6 Wasserstoff einen gegebenenfalls substituierten C₁-C₈-Alkyl-, oder einen gegebenenfalls substituierten monocyclischen oder bicyclischen Cycloalkyl-, Aryl-, Heteroaryl-, Aralkyl- oder Alkylheteroaryl-Rest,
oder
R5 und R6 zusammen mit dem N-Atom einen gesättigten oder ungesättigten 5- oder 6-gliedrigen Ring, der maximal ein zusätzliches Heteroatom beinhaltet,
bedeutet,
deren pharmakologisch verträgliche Salze, Ester und Derivate, die zu in vivo zu Verbindungen der allgemeinen Formel I metabolisiert werden.

2. Verbindungen der Formel I 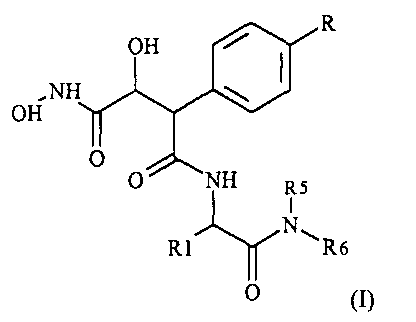 in denen
R als Ph-R-Fragment einen clogP-Wert zwischen 2.0 und 6.0 aufweist,
R1 als Aminocarbonyl-R1-Fragment einen clogP-Wert zwischen -1.5 und 2.0 aufweist, und
R5 Wasserstoff, oder einen C₁-C₈-Alkyl-Rest,
R6 Wasserstoff, einen gegebenenfalls substituierten C₁-C₈-Alkyl-, oder einen gegebenenfalls substituierten monocyclischen oder bicyclischen Cycloalkyl, Aryl-, Heteroaryl-, Aralkyl- oder Alkylheteroaryl-Rest,
oder
R5 und R6 zusammen mit dem N-Atom einen gesättigten oder ungesättigten 5- oder 6-gliedrigen Ring, der maximal ein zusätzliches Heteroatom beinhaltet,
bedeutet,
deren pharmakologisch verträgliche Salze, Ester und Derivate, die zu in vivo zu Verbindungen der allgemeinen Formel I metabolisiert werden.

3. Verbindungen der Formel I nach einem der Ansprüche 1 oder 2, in denen der Rest R 4 bis 7 Gerüstatome bei Alkyl oder Cycloalkyl oder 5 bis 10 Gerüstatome bei Aryl, Bicyclus oder Heteroaryl aufweist.

4. Verbindungen der Formel I nach einem der Ansprüche 1 bis 3, in denen der Rest R1 3 bis 10 Gerüstatome aufweist.

5. Verbindungen der Formel I nach einem der Ansprüche 1 bis 4, in denen der Rest R5 Wasserstoff, Methyl und Ethyl bedeutet.

6. Verbindungen der Formel I nach einem der Ansprüche 1 bis 5, in denen der Rest R6 Methyl und Ethyl, Phenyl oder Pyridyl bedeutet.

7. Verbindungen der Formel I nach einem der Ansprüche 1 bis 4, in denen die Reste R5 und R6 einen Zyklus bedeuten der Sauerstoff enthält.

8. Verbindungen der Formel I nach einem der Ansprüche 1 bis 7, die als optische Isomere in der S-Konfiguration bei C2 der Bernsteinhydroxamsäure und Cα des Aminosäureamids und in der R-Konfiguration an C3 des Bernsteinsäuregrundkörpers vorliegen.

9. Arzneimittel, enthaltend mindestens eine Verbindung der Formel I gemäß einem der Ansprüche 1 bis 8 neben üblichen Träger- und Hilfsstoffen.

10. Verwendung von Verbindungen der Formel I gemäß einem der Ansprüche 1 bis 8 zur Herstellung von Arzneimitteln mit Gelatinase-inhibierender Wirkung.
